# EUROPÄISCHE PATENTSCHRIFT

(11) **EP 0 962 447 B1**
(45) Veröffentlichungstag und Bekanntmachung des Hinweises auf die Patenterteilung: **18.12.2002**
(21) Anmeldenummer: 99110687.3
(22) Anmeldetag: 02.06.1999
(51) Int. Cl.: C07C 209/48, C07C 211/09

(54) **Verfahren zur Herstellung von sekundären bzw. tertiären 2-Methyl-1,5-pentandiaminen**
Process for the preparation of secondary or tertiary 2-methyl-1,5-pentanediamines
Procédé pour la préparation de 2-méthylpentane-1,5-diamines secondaires ou tertiaires

(30) Priorität: 06.06.1998 DE 19825452
(43) Veröffentlichungstag der Anmeldung: 08.12.1999
(73) Patentinhaber: BASF AKTIENGESELLSCHAFT, 67056 Ludwigshafen (DE)
(72) Erfinder: Eller, Karsten Dr., 67061 Ludwigshafen (DE); Fiege, Bernd, 67227 Frankenthal (DE); Rittinger, Stefan Dr., 68199 Mannheim (DE); Fuchs, Eberhard Dr., 67227 Frankenthal (DE)

(56) Entgegenhaltungen:
- EP-A- 0 599 180
- EP-A- 0 869 113
- WO-A-95/30666
- WO-A-96/33986
- DE-A- 19 614 283
- GB-A- 1 157 637
- US-A- 3 673 251
- US-A- 4 288 626
- L. CERVENY: STUDIE IN SURFACE SCIENCE AND CATALYSIS, Bd. 27, 1986, Seite 122-125,140 XP000926686 Elsevier

## Beschreibung

Die Erfindung betrifft ein Verfahren zur Herstellung von sekundären bzw. tertiären 2-Methyl-1,5-pentandiaminen der Formel I 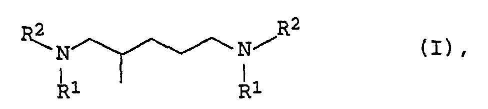 in der
- R¹ und R²: H, C₁₋₂₀-Alkyl, C₃₋₈-Cycloalkyl, Aryl, C₇₋₂₀-Aryl-alkyl,
wobei die Reste R¹ und R² Substituenten, ausgewählt aus der Gruppe C₁₋₂₀-Alkyl, C₁₋₂₀-Alkoxy, C₆₋₂₀-Aryloxy, Hydroxy, tragen können sowie R¹ und R² nicht gleichzeitig Wasserstoff darstellen,
bedeuten,
oder
- R¹ und R²: gemeinsam eine gegebenenfalls durch C₁₋₂₀-Alkyl und/oder C₁₋₂₀-Alkoxy substituierte C₃₋₇-Alkylenkette, die gegebenenfalls ein- oder zweifach durch O oder NR³ unterbrochen sein kann, und
- R³: H, C₁₋₂₀-Alkyl
bedeuten.

Weiterhin betrifft die Erfindung die Verbindung 1,5-Bis-(dimethylamino)-2-methyl-pentan.

In der US 3,673,251 ist ein kontinuierliches Verfahren zur Herstellung von sekundären und/oder tertiären Mono-, Di- oder Polyaminen aus Mono-, Di- oder Polynitrilen und primären bzw. sekundären Aminen beschrieben. Bei der Herstellung von N,N,N',N'-Tetraethyl-2-methyl-1,5-pentandiamin (loc. cit., Beispiel 3) wird in einem aufwendigem Verfahren unter Verwendung von 5 % Palladium auf Kohle und Diatomeenerde als Katalysator und unter Adsorption des entstehenden Ammoniaks an festem Calciumchlorid eine Ausbeute von 60 % erzielt.

Die WO 95/30666 offenbart die Selbstkondensation von drei Molekülen 2-Methylglutarsäuredinitril zu 1,5-Bis-(3-methylpiperidino)-2-methyl-pentan in Gegenwart von Wasserstoff an Pd/Al₂O₃-Katalysatoren. Dieses Verfahren ist jedoch nicht auf die Herstellung anderer tertiärer oder sekundärer 2-Methyl-1,5-pentandiamine übertragbar.
Beispiel 8 dieser Patentanmeldung beschreibt die Umsetzung von überschüssigem 3-Methylpiperidin mit 2-Methylglutarsäuredinitril und Wasserstoff in Gegenwart eines Pd/Al₂O₃-Katalysators, wobei als Hauptprodukt 1,5-Bis-(3-methylpiperidino)-2-methyl-pentan entsteht. Vor der Umsetzung war im sehr kleinen und mit Katalysator gefüllten Reaktor (Katalysatormenge: 3 g) im Wasserstoffstrom unter Druck die Reaktionstemperatur von 180°C eingestellt worden.

Die WO 96/33986 beschreibt die Umsetzung von 2-Methylglutarsäuredinitril mit 3-Methylpiperidin und Wasserstoff an einem Pd/Al₂O₃-Katalysator zu einem Gemisch aus 2- und 4-Methyl-5-(3-methylpiperidino)-pentannitril, welches in einem separaten Schritt zu 2- und 4-Methyl-5-(3-methylpiperidino)-pentan-1-amin hydriert wird. Das entsprechende tertiäre Diamin 1,5-Bis-(3-methylpiperidino)-2-methyl-pentan wird dabei nur als Nebenprodukt in Ausbeuten von maximal 24,4 % erhalten (loc. cit., Beispiel 1). Vor der Umsetzung war der sehr kleine und mit Katalysator gefüllte Reaktor (Katalysatormenge: 3 g) im Wasserstoffstrom unter Druck auf die Reaktionstemperatur von 150°C aufgeheizt worden.

GB-A-1,157,637, GB-A-1,157,638 und GB-A-1,157,639 offenbaren die Umsetzung von 2-Methylglutarsäuredinitril mit Diethylamin in Gegenwart von Wasserstoff und Palladium zu 5-Diethylamino-2-methylvaleronitril.
Trotz langer Reaktionszeiten und hohen molaren Überschüssen an Diethylamin wurde dabei kein N,N,N',N'-Tetraethyl-2-methyl-1,5-pentandiamin gefunden.

Gemäß EP-A-673 918 werden Nitrile mit sekundären Aminen und Wasserstoff in Gegenwart von Palladium-Katalysatoren zu peralkylierten Aminen umgesetzt. Dabei werden 3-Dimethylamino-propionitril und Dimethylamin zu N,N,N',N'-Tetramethylpropylen-1,3-diamin, 3-Hydroxypropionitril und Dimethylamin zu 3-Dimethylamino-propan-1-ol und Piperazin mit Acetonitril zu N-Ethylpiperazin umgesetzt.

In der EP-A-599 180 ist ein Verfahren zur Herstellung von N,N,N',N'-tetrasubstituierten Diaminen durch Umsetzung von Dinitrilen mit sekundären Aminen beschrieben. Dabei wird Adipodinitril mit Dimethylamin und Wasserstoff in Gegenwart eines Pd/Al₂O₃-Katalysators zu N,N,N',N'-Tetramethyl-hexamethylendiamin umgesetzt (loc. cit., Beispiele 1 bis 6).
Beim Versuch, das Verfahren gemäß EP-A-599 180 auf die Herstellung von N,N,N',N'-2-Pentamethylpentan-1,5-diamin aus 2-Methylglutarsäuredinitril und Dimethylamin zu übertragen, werden nur unbefriedigende Ausbeuten erzielt.

Eine Abhängigkeit der Ergebnisse der Umsetzung von Nitrilen mit Aminen und Wasserstoff in Gegenwart von Edelmetall enthaltenden Katalysatoren von der Molekülstruktur des eingesetzten Nitrils wird in 'L. Cerveny, Studies in Surface Science and Catalysis, Vol. 27: Catalytic Hydrogenation, Seite 122, Zeilen 1-4, (Elsevier, 1986)' erwähnt.

Die ältere deutsche Patentanmeldung Nr. 197 13 383.5 beschreibt ein gegenüber der EP-A-673 918 und der EP-A-599 180 verbessertes Verfahren (vergl. loc. cit., Seite 2, Zeilen 1-2) zur Herstellung von tertiären Aminen aus Nitrilen und sekundären Aminen und Wasserstoff in Gegenwart eines Palladium enthaltenden Katalysators.
Gemäß des Beispiels wird aus Adipodinitril und Dimethylamin unter Verwendung eines verbesserten Katalysators, bestehend aus Palladium und Platin auf Zirkondioxid, N,N,N',N'-Tetramethylhexamethylendiamin hergestellt.

'L. Cerveny, Studies in Surface Science and Catalysis, Vol. 27: Catalytic Hydrogenation' lehrt auf Seite 124, letzter Absatz, Seite 125, erster Absatz, und Seite 140, Kapitel 4.7, Zeile 10-11, daß die Beschaffenheit des Trägers des Edelmetallkatalysators bei der Umsetzung von Nitrilen mit Aminen nur einen geringen Einfluß auf die Katalysatoreigenschaften hat. Gemäß loc. cit., Seite 122, vorletzter Absatz, wird ein geträgerter Palladium-Katalysator durch eine Vorbehandlung bei 200°C mit Wasserstoff inaktiv für die katalytische Umsetzung von Nitrilen zu primären, sekundären oder tertiären Aminen.

Der Erfindung lag die Aufgabe zugrunde, ein wirtschaftliches Verfahren zu finden, das es erlaubt, sekundäre bzw. tertiäre 2-Methyl-1,5-pentandiamine ausgehend von 2-Methylglutarsäuredinitril und primären bzw. sekundären Aminen in guten Ausbeuten und Selektivitäten und bei langen Katalysatorstandzeiten zu erhalten. Eine weitere Aufgabe bestand in der Bereitstellung von 1,5-Bis-(dimethylamino)-2-methyl-pentan.

Demgemäß wurde ein Verfahren zur Herstellung von sekundären bzw. tertiären 2-Methyl-1,5-pentandiaminen der Formel I 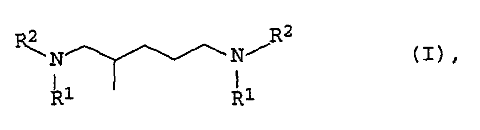 in der R¹ und R² die eingangs angegebene Bedeutung haben, gefunden, wobei man 2-Methylglutarsäuredinitril 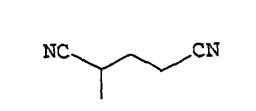 mit einem primären bzw. sekundären Amin der Formel R¹R²NH und Wasserstoff bei Temperaturen von 50 bis 250°C und Drücken von 0,5 bis 35 MPa in Gegenwart eines ein oder mehrere Edelmetalle enthaltenden oxidischen Trägerkatalysators, der vor seinem Einsatz bei Temperaturen von 50 bis 300°C über einen Zeitraum von mindestens 0,5 Stunden mit Wasserstoff behandelt wurde, umsetzt.

Weiterhin wurde 1,5-Bis-(dimethylamino)-2-methyl-pentan gefunden.

Die Reste R¹ und R² haben unabhängig voneinander folgende Bedeutungen, wobei R¹ und R² nicht gleichzeitig Wasserstoff darstellen:
- Wasserstoff (H),
- C₁₋₂₀-Alkyl, vorzugsweise C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl, bevorzugt C₁₋₄-Alkyl, wie Methyl, Ethyl, n-Propyl, iso-Propyl, n-Butyl, iso-Butyl, sec.-Butyl und tert.-Butyl, besonders bevorzugt Methyl,
- C₃₋₈-Cycloalkyl, wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
   besonders bevorzugt Cyclopentyl und Cyclohexyl,
- Aryl, bevorzugt C₆₋₂₀-Aryl, wie Phenyl, 1-Naphthyl, 2-Naphthyl, 1-Anthryl, 2-Anthryl, 9-Anthryl, besonders bevorzugt Phenyl, 1-Naphthyl und 2-Naphthyl, ganz besonders bevorzugt Phenyl,
- C₇₋₂₀-Arylalkyl, wie Benzyl, 1-Phenethyl, 2-Phenethyl, 1-Naphthylmethyl, 2-Naphthylmethyl, Phenanthrylmethyle, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl und 4-Phenylbutyl,
   besonders bevorzugt Benzyl, 1-Phenethyl und 2-Phenethyl,
wobei in diesen Fällen die Reste R¹ und R² unabhängig voneinander unter den Reaktionsbedingungen inerte Substituenten tragen können, wie z. B. C₁₋₂₀-Alkyl, C₁₋₂₀-Alkoxy, C₆₋₂₀-Aryloxy und Hydroxy.

Dabei kann die Anzahl dieser Substituenten in R¹ und R² in Abhängigkeit von der Art des Restes 0 bis 5, vorzugsweise 0 bis 3, insbesondere 0, 1 oder 2 betragen. Als Substituenten kommen in Betracht:
- C₁₋₂₀-Alkyl, wie oben definiert,
- C₁₋₂₀-Alkoxy, bevorzugt C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy,
   besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy,
- C₆₋₂₀-Aryloxy, wie Phenoxy, 1-Naphthoxy und 2-Naphthoxy, bevorzugt Phenoxy,
- Hydroxy (-OH).

Weiterhin können die Reste R¹ und R² neben der obigen Definition auch gemeinsam eine C₃₋₇-Alkylenkette,
wie -(CH₂)₃-, -(CH₂)₄-, -(CH₂)₅-, -(CH₂)₆-, -(CH₂)₇-, bevorzugt -(CH₂)₄- und -(CH₂)₅-,
die gegebenenfalls ein- oder zweifach durch O oder NR³ unterbrochen sein kann,
wie beispielsweise - (CH₂)-O-(CH₂)₂-, -(CH₂)-NR³-(CH₂)₂-, -(CH₂)₂-O-(CH₂)₂-, -(CH₂)₂-NR³-(CH₂)₂-, -(CH₂)₂-O-(CH₂)₃-, -CH₂-NR³-(CH₂)₃-, bevorzugt - (CH₂)₂-O-(CH₂)₂- und -(CH₂)₂-NR³-(CH₂)₂-,
und die zusätzlich ein oder zwei unter den Reaktionsbedingungen inerte Substituenten tragen kann, wie zum Beispiel C₁₋₂₀-Alkyl und/oder C₁₋₂₀-Alkoxy,
mit C₁₋₂₀-Alkyl und C₁₋₂₀-Alkoxy wie oben definiert,
wie beispielsweise -CH₂-CHCH₃-O-CHCH₃-CH₂-, -(CH₂)₂-CH(OCH₃)-(CH₂)₂-, -(CH₂)₂-CH(OC₂H₅)-(CH₂)₂-, -(CH₂)-CHCH₃-(CH₂)₂-, -(CH₂)₂-CHCH₃-(CH₂)₂-, -(CH₂)-CHCH₃-(CH₂)₃-, - (CH₂)-CHCH₃-(CH₂)₂-,
bedeuten.

Der Rest R³ hat folgende Bedeutungen:
- Wasserstoff (H),
- C₁₋₂₀-Alkyl, wie oben definiert.

Die Verfahren lassen sich wie folgt ausführen:

Zur Herstellung von tertiären Aminen der Formel R¹R²N-CH₂-CHCH₃-(CH₂)₃-NR¹R² wird 2-Methylglutarsäuredinitril mit einem sekundären Amin der Formel R¹R²NH in Gegenwart von Wasserstoff und einem ein oder mehrere Edelmetalle enthaltenden oxidischen Trägerkatalysator nach dem Verfahren gemäß Anspruch 1 umgesetzt.

Zur Herstellung von sekundären Aminen der Formel R¹HN-CH₂-CHCH₃-(CH₂)₃-NHR¹ wird 2-Methylglutarsäuredinitril mit einem primären Amin der Formel R¹NH₂ (d.h. R² ist hier gleich H) in Gegenwart von Wasserstoff und einem ein oder mehrere Edelmetalle enthaltenden oxidischen Trägerkatalysator nach dem Verfahren gemäß Anspruch 1 umgesetzt.

Der Trägerkatalysator wird erfindungsgemäß vor seinem Einsatz zur Aminierung des Nitrils mit Wasserstoff behandelt. Dies geschieht zum Beispiel dadurch, daß der bereits im Reaktor eingebaute Trägerkatalysator mit Wasserstoffgas oder einem Wasserstoff enthaltenden Inertgasstrom (z.B. Stickstoff, Argon) gespült wird. Reines Wasserstoffgas wird hierbei bevorzugt.

Bei der Behandlung des Katalysators mit Wasserstoff ist jeder der Einsatzstoffe, 2-Methylglutarsäuredinitril und primäres bzw. sekundäres Amin, abwesend.

Die Behandlung des Trägerkatalysators mit Wasserstoff geschieht in der Regel bei Drücken von 0,1 bis 35 MPa. Sie kann auch bei höheren Drücken, z.B. 50 MPa, erfolgen, jedoch erfordert dies einen unnötig hohen Aufwand von apparatetechnischer Seite. Bevorzugt sind Drücke von 0 bis 25 MPa, besonders bevorzugt 0 bis 20 MPa.

Im allgemeinen erfolgt die Behandlung des Trägerkatalysators mit Wasserstoff bei Temperaturen von 50 bis 300°C, bevorzugt 80 bis 250°C, besonders bevorzugt 100 bis 200°C, und ganz besonders bevorzugt 130 bis 160°C.

In vielen Fällen kann man praktischerweise so vorgehen, daß man in dem Reaktor in dem auch die spätere Aminierungsreaktion vorgenommen wird und bei den später in dieser Reaktion angewandten Drücken und Temperaturen auch die Behandlung des Trägerkatalysators mit Wasserstoff vornimmt.

Die Behandlungsdauer des Trägerkatalysators mit Wasserstoff beträgt mindestens 0,5 Stunden, bevorzugt mindestens 2 Stunden, besonders bevorzugt mindestens 3 Stunden. Längere Behandlungsdauern als 2 Tage bewirken in der Regel keine weiteren Verbesserungen der Katalysatoreigenschaften.

Je nach gewählter Temperatur und gewähltem Wasserstoffdruck liegt die Behandlungsdauer des Trägerkatalysators mit Wasserstoff in der Regel im Bereich von 0,5 bis 48 Stunden, bevorzugt 2 bis 24 Stunden.

Beispielsweise kann bei Wasserstoff-Drücken von 0 bis 25 MPa und Temperaturen von 100 bis 200°C die Behandlungsdauer bei 2 bis 24, vorzugsweise 4 bis 12, Stunden liegen. Im allgemeinen sinken die erforderlichen Behandlungsdauern mit zunehmendem Druck und zunehmender Temperatur.

Als Katalysator für das erfindungsgemäße Verfahren eignen sich ein oder mehrere Edelmetalle auf oxidischen Trägern. Als Edelmetalle können insbesondere Silber, Rhenium, Ruthenium, Palladium, Platin, Rhodium, Osmium oder Iridium verwendet werden. Bevorzugt sind Palladium, Platin und Ruthenium, besonders bevorzugt ist die gleichzeitige Verwendung von Palladium und Platin.

Die Gesamtmenge an Edelmetallen, berechnet als Gesamtmenge an Edelmetallen mit der Oxidationsstufe 0, im Trägerkatalysator liegt bei 0,01 bis 10 Gew.-%, bevorzugt 0,01 bis 3 Gew.-%, besonders bevorzugt 0,01 bis 1,5 Gew.-%, bezogen auf das Gesamtgewicht des Katalysators.

Im allgemeinen enthalten die Katalysatoren jeweils 0,01 bis 5 Gew.-%, bevorzugt 0,01 bis 1 Gew.-%, jedes Edelmetalls. Bevorzugt sind Katalysatoren enthaltend 0,1 bis 1 Gew.-% Palladium. Besonders bevorzugt sind Katalysatoren enthaltend 0,1 bis 1 Gew.-% Palladium und zusätzlich 0,01 bis 1 Gew.-% Platin.

Zusätzlich zu den Edelmetallen können die Katalysatoren noch mit anderen Metallen dotiert sein, so beispielsweise mit anderen Übergangsmetallen oder Lanthaniden.

Geeignete Katalysatoren wurden zum Beispiel in EP-A-599 180, EP-A-673 918 und der deutschen Patentanmeldung Nr. 197 13 383.5 beschrieben.

Als Träger können insbesondere alle oxidischen Träger eingesetzt werden. Bevorzugt sind γ- oder α-Aluminiumoxid, Siliciumdioxid, Titandioxid, Zirkondioxid, Cerdioxid, mit Alkali- oder Erdalkalioxiden dotiertes Al₂O₃, SiO₂, TiO₂ oder ZrO₂, oder deren Gemische. Besonders bevorzugt sind Aluminiumoxid und Zirkondioxid. Ganz besonders bevorzugt ist Zirkondioxid. Die Träger können in beliebiger Form eingesetzt werden, beispielsweise als Pulver, Stränge, Tabletten, Kugeln oder Ringe. Die Herstellung der Träger und die Aufbringung der Edelmetallkomponenten kann nach den üblichen Verfahren erfolgen, wie zum Beispiel in EP-A-599 180 und EP-A-673 918 beschrieben.

Exemplarisch soll hier nur eine Tränkung mit einer Lösung der Edelmetalle in Form ihrer Nitrate, Acetate oder Chloride gefolgt von Trocknung, Kalzinierung und eventueller Reduktion genannt werden. Bei Aufbringung mehrerer Edelmetalle kann die Tränkung gleichzeitig oder in beliebiger Reihenfolge nacheinander erfolgen.

Die beiden Edukte 2-Methylglutarsäuredinitril und primäres bzw. sekundäres Amin werden im erfindungsgemäßen Verfahren üblicherweise in einem molaren Verhältnis von 1 : 2 bis 1: 40, bevorzugt 1 : 3 bis 1: 20, besonders bevorzugt 1 : 4 bis 1 : 15 eingesetzt. Es können jedoch auch größere Aminüberschüsse verwendet werden, da im allgemeinen eine Rückführung nicht umgesetzten Amins aufgrund der unterschiedlichen Siedepunkte leicht möglich ist.

Beispiele für einsetzbare primäre Amine sind Methylamin, Ethylamin, n- und i-Propylamin, n-Butylamin, i-Butylamin, tert.-Butylamin, n-Pentylamin, i-Pentylamin, neo-Pentylamin, n-Hexylamin, 2-Ethylhexylamin, Cyclopropylamin, Cyclopentylamin, Cyclohexylamin, 2-Methylpentylamin, 2-Methylhexylamin, 4-Methylhexylamin, Anilin, 2,6-Dimethylanilin, Ethanolamin und 2-Phenoxyethylamin. Bevorzugt sind Methylamin, Ethylamin, n-Propylamin, i-Propylamin, Cyclohexylamin, besonders bevorzugt Methylamin und Ethylamin.

Beispiele für einsetzbare sekundäre Amine sind Dimethylamin, Diethylamin, Methylethylamin, Di-n-propylamin, Diisopropylamin, Di-n-butylamin, Diisobutylamin, Di-s-butylamin, Ethylisopropylamin, Bis-(2-ethylhexyl)amin, Dicyclohexylamin, Methylcyclohexylamin, Diphenylamin, N-Ethyl-anilin, Diethanolamin, Pyrrolidin, Piperidin, 2-Methylpiperidin, 3-Methylpiperidin, Morpholin, Piperazin, 1-Methylpiperazin und 1-Ethylpiperazin. Bevorzugt sind Dimethylamin, Diethylamin, Di-n-butylamin, Piperidin und Morpholin, besonders bevorzugt sind Dimethylamin und Diethylamin. Ganz besonders bevorzugt ist Dimethylamin.

Die Umsetzung des 2-Methylglutarsäuredinitrils mit dem primären bzw. sekundären Amin kann kontinuierlich oder diskontinuierlich erfolgen. Bei einer diskontinuierlichen Durchführung, z. B. ein einem Druckautoklaven, werden beispielsweise die Edukte zusammen mit dem Trägerkatalysator, der wie oben beschrieben zuvor mit Wasserstoff behandelt wurde, vorgelegt, Wasserstoff aufgepreßt und dann auf Reaktionstemperatur gebracht. Nach Beendigung der Reaktion wird das Reaktionsgefäß entspannt und der Reaktorinhalt aufgearbeitet. Der Katalysator kann hierbei als Pulver oder in einer geeigneten Vorrichtung auch in Form von Strängen, Tabletten, etc. eingesetzt werden.

Bevorzugt wird die Reaktion kontinuierlich in einer Druckbehälterkaskade oder in einem Reaktor, in dem der Katalysator als Festbett angeordnet ist, wie z. B. einem Rohrreaktor, durchgeführt. Die Katalysatorbelastung liegt dabei üblicherweise im Bereich von 0,05 bis 1 l_{Nitril} • l_{Kat.}⁻¹ • h⁻¹, bevorzugt im Bereich von 0,1 bis 0,8 l_{Nitril} • l_{Kat.}⁻¹ • h⁻¹.

Die Reaktionstemperatur bei der Umsetzung des 2-Methylglutarsäuredinitrils mit dem primären bzw. sekundären Amin richtet sich nach der Reaktivität des Amins sowie der gewählten Belastung des Katalysators. Geeignete Reaktionstemperaturen liegen im Bereich von 50 bis 250°C, bevorzugt 90 bis 200°C, besonders bevorzugt 100 bis 180°C, ganz besonders bevorzugt 110 bis 170°C.

Der Wasserstoffdruck bei der Umsetzung des 2-Methylglutarsäuredinitrils mit dem primären bzw. sekundären Amin liegt im Bereich zwischen 0,5 und 35 MPa, bevorzugt zwischen 5 und 30 MPa, besonders bevorzugt zwischen 10 und 25 MPa.

Wahlweise kann die Reaktion auch in einem inerten Lösungsmittel wie Tetrahydrofuran (THF) oder N-Methyl-pyrrolidon (NMP) durchgeführt werden. Im Lösungsmittel kann dabei das 2-Methylglutarsäuredinitril und/oder das primäre bzw. sekundäre Amin und/oder bei der Umsetzung entstehender Ammoniak gelöst sein. Vorzugsweise wird ohne Lösungsmittel gearbeitet.

Die im erfindungsgemäßen Verfahren erhaltenen sekundären bzw. tertiären Amine der Formel I lassen sich in an sich bekannter Weise vom Reaktionsaustrag abtrennen und reinigen, beispielsweise destillativ.

Die Amine der Formel I sind wichtige Zwischenprodukte für die Herstellung von Effektstoffen, wie Farbstoffen, und Wirkstoffen und eignen sich als Katalysatoren für die Herstellung von Polyurethanen sowie als Emulgatoren, Weichmacher, Korrosionsinhibitoren und Textilhilfsmittel.

### Beispiele

Alle Angaben für Produktzusammensetzungen erfolgen in GC-Flächen-%.

### Beispiel 1

In einen Rohrreaktor (Länge 1450 mm, Durchmesser 30 mm) wurden 500 ml eines Katalysators eingebaut, der aus 0,5 Gew.-% Palladium auf 4 mm Aluminiumoxidsträngen bestand. Die Reduktion erfolgte bei 140 °C und 20 MPa H₂ für 4 h. Anschließend wurden bei 140°C und 20 MPa Wasserstoffdruck stündlich 75 ml 2-Methylglutarsäuredinitril und 200 g Dimethylamin (als Flüssiggas, entsprechend 294 ml) in Sumpffahrweise über den Katalysator geleitet (Molverhältnis 1 : 6,7). Gleichzeitig wurden 2,5 l pro h des Reaktoraustrags in den Reaktor zurückgeführt (Umlauffahrweise). Ein typischer Reaktoraustrag enthielt unter diesen Bedingungen nach Abtrennung von Ammoniak und überschüssigem Dimethylamin durch Entgasen nach gaschromatographischer Analyse
84,7 % N,N,N',N'-2-Pentamethyl-pentan-1,5-diamin,
0 % 2-Methylglutarsäuredinitril
sowie als Nebenprodukte
4,3 % N,N,N'-2-Tetramethyl-pentan-1,5-diamin und N,N',N'-2-Tetramethyl-pentan-1,5-diamin,
4,3 % N',N' -2-Trimethyl-pentan-1,5-diamin und N,N-2-Trimethylpentan-1,5-diamin und
6,7 % sonstige Verbindungen.

### Beispiel 2

Eine Durchführung des Versuchs wie im Beispiel 1 beschrieben, mit dem Unterschied, daß der Reaktor nicht in Umlauffahrweise sondern im geraden Durchgang betrieben wurde, ergab nach der Abtrennung von Ammoniak und überschüssigem Dimethylamin einen Reaktoraustrag mit einem Gehalt von
78,4 % N,N,N',N'-2-Pentamethyl-pentan-1,5-diamin,
0 % 2-Methylglutarsäuredinitril
sowie als Nebenprodukten
6,8 % N,N,N'-2-Tetramethyl-pentan-1,5-diamin und N,N',N'-2-Tetramethyl-pentan-1,5-diamin,
5,3 % N',N'-2-Trimethyl-pentan-1,5-diamin und N,N-2-Trimethylpentan-1,5-diamin und
9,5 % sonstigen Verbindungen.

### Beispiel 3

In einen Rohrreaktor (Länge 1450 mm, Durchmesser 30 mm) wurden 500 ml eines Katalysators eingebaut, der aus 0,9 Gew.-% Palladium und 0,1 Gew.-% Platin auf 4 mm Zirkondioxidsträngen bestand. Die Reduktion erfolgte bei 140 °C mit 100 l/h H₂ drucklos für 4 h. Anschließend wurden bei 120 °C und 20 MPa Wasserstoffdruck stündlich 75 ml 2-Methylglutarsäuredinitril und 200 g Dimethylamin (als Flüssiggas, entsprechend 294 ml) in Sumpffahrweise über den Katalysator geleitet (Molverhältnis 1 : 6,7). Die Temperatur wurde danach schrittweise auf 150 °C erhöht. Ein typischer Reaktoraustrag enthielt unter diesen Bedingungen nach Abtrennung von Ammoniak und überschüssigem Dimethylamin nach gaschromatographischer Analyse
83,4 % N,N,N',N'-2-Pentamethyl-pentan-1,5-diamin,
0 % 2-Methylglutarsäuredinitril
sowie als Nebenprodukte
5,6 % N,N,N'-2-Tetramethyl-pentan-1,5-diamin und N,N',N'-2-Tetramethyl-pentan-1,5-diamin,
4,3 % N',N'-2-Trimethyl-pentan-1,5-diamin und N,N-2-Trimethylpentan-1,5-diamin und
6,7 % sonstige Verbindungen.

### Beispiel 4

Eine Durchführung des Versuchs wie im Beispiel 3 beschrieben, mit dem Unterschied, daß die Reaktion bei einer Reaktortemperatur von 160 °C durchgeführt wurde, ergab nach der Abtrennung von Ammoniak und überschüssigem Dimethylamin einen Reaktoraustrag mit einem Gehalt von
84,4 % N,N,N',N'-2-Pentamethyl-pentan-1,5-diamin,
0 % 2-Methylglutarsäuredinitril
sowie als Nebenprodukten
5,5 % N,N,N'-2-Tetramethyl-pentan-1,5-diamin und N,N',N'-2-Tetramethyl-pentan-1,5-diamin,
4,1 % N',N'-2-Trimethyl-pentan-1,5-diamin und N,N-2-Trimethylpentan-1,5-diamin und
6,0 % sonstigen Verbindungen.

### Beispiel 5

Eine Durchführung des Versuchs wie im Beispiel 4 beschrieben, mit dem Unterschied, daß die Reaktion bei einem Molverhältnisses von 2-Methylglutarsäuredinitril : Dimethylamin = 1 : 10.1 durchgeführt wurde, ergab keine Veränderung der Ausbeute an N,N,N',N'-2-Pentamethyl-pentan-1,5-diamin. Der Versuch wurde nach 42 Tagen beendet, ohne daß eine Desaktivierung des Katalysators beobachtet worden wäre.

### Beispiel 6

In einen Rohrreaktor (Länge 1450 mm, Durchmesser 30 mm) wurden 500 ml eines Katalysators eingebaut, der aus 0,5 Gew.-% Palladium auf 3 mm Zirkondioxidtabletten bestand. Die Reduktion erfolgte bei 140 °C und 20 MPa H₂ für 10 h. Anschließend wurden bei 150°C und 20 MPa Wasserstoffdruck stündlich 75 ml 2-Methylglutarsäuredinitril und 200 g Dimethylamin (als Flüssiggas, entsprechend 294 ml) in Sumpffahrweise über den Katalysator geleitet (Molverhältnis 1 : 6.7). Die Temperatur wurde dann auf 160°C erhöht. Ein typischer Reaktoraustrag enthielt unter diesen Bedingungen nach Abtrennung von Ammoniak und überschüssigem Dimethylamin nach gaschromatographischer Analyse
76,8 % N,N,N',N'-2-Pentamethyl-pentan-1,5-diamin,
0 % 2-Methylglutarsäuredinitril
sowie als Nebenprodukte
6,6 % N,N,N'-2-Tetramethyl-pentan-1,5-diamin und N,N',N'-2-Tetramethyl-pentan-1,5-diamin,
5,4 % N',N'-2-Trimethyl-pentan-1,5-diamin und N,N-2-Trimethylpentan-1,5-diamin und
11,2 % sonstige Verbindungen.

## Patentansprüche

1. Verfahren zur Herstellung von sekundären bzw. tertiären 2-Methyl-1,5-pentandiaminen der Formel I 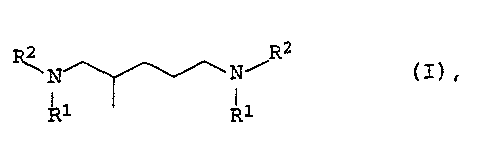 in der
R¹ und R² H, C₁₋₂₀-Alkyl, C₃₋₈-Cycloalkyl, Aryl, C₇₋₂₀-Aryl-alkyl,
wobei die Reste R¹ und R² Substituenten, ausgewählt aus der Gruppe C₁₋₂₀-Alkyl, C₁₋₂₀-Alkoxy, C₆₋₂₀-Aryloxy, Hydroxy, tragen können sowie R¹ und R² nicht gleichzeitig Wasserstoff darstellen,
bedeuten,
oder
R¹ und R² gemeinsam eine gegebenenfalls durch C₁₋₂₀-Alkyl und/oder C₁₋₂₀-Alkoxy substituierte C₃₋₇-Alkylenkette, die gegebenenfalls ein- oder zweifach durch O oder NR³ unterbrochen sein kann, und
R³ H, C₁₋₂₀-Alkyl
bedeuten,
wobei man 2-Methylglutarsäuredinitril mit einem primären bzw. sekundären Amin der Formel R¹R²NH und Wasserstoff bei Temperaturen von 50 bis 250°C und Drücken von 0,5 bis 35 MPa in Gegenwart eines ein oder mehrere Edelmetalle enthaltenden oxidischen Trägerkatalysators, der vor seinem Einsatz bei Temperaturen von 50 bis 300°C über einen Zeitraum von mindestens 0,5 Stunden mit Wasserstoff behandelt wurde, umsetzt.

2. Verfahren nach Anspruch 1, wobei der Trägerkatalysator vor seinem Einsatz bei Temperaturen von 50 bis 300 °C über einen Zeitraum von mindestens 2 Stunden mit Wasserstoff behandelt wurde.

3. Verfahren nach den Ansprüchen 1 und 2, wobei der Trägerkatalysator die Edelmetalle Palladium und/oder Platin und/oder Ruthenium enthält.

4. Verfahren nach den Ansprüchen 1 bis 3, wobei der Trägerkatalysator 0,1 bis 5 Gew.-% Palladium und 0,01 bis 5 Gew.-% Platin enthält.

5. Verfahren nach den Ansprüchen 1 bis 4, wobei man als Träger für den Katalysator Zirkondioxid oder Aluminiumoxid verwendet.

6. Verfahren nach den Ansprüchen 1 bis 5, wobei man als Amin Dimethylamin einsetzt.

7. 1,5-Bis-(dimethylamino)-2-methyl-pentan.

## Claims

1. A process for preparing a secondary or tertiary 2-methyl-1,5-pentanediamine of the formula I 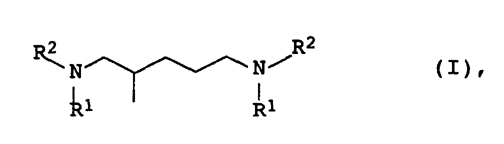 where
R¹ and R² are H, C₁-C₂₀-alkyl, C₃-C₈-cycloalkyl, aryl, C₇-C₂₀-arylalkyl,
where the radicals R¹ and R² may bear substituents selected from the group consisting of C₁-C₂₀-alkyl, C₁-C₂₀-alkoxy, C₆-C₂₀-aryloxy and hydroxy, and R¹ and R² are not simultaneously hydrogen,
or
R¹ and R² are together an unsubstituted or C₁-C₂₀-alkyl-and/or C₁-C₂₀-alkoxy-substituted C₃-C₇-alkylene chain which may, if desired, be interrupted by one or two O or NR³ groups, where
R³ is H or C₁-C₂₀-alkyl
which comprises reacting 2-methylglutarodinitrile with a primary or secondary amine of the formula R¹R²NH and hydrogen at from 50 to 250°C and pressures of from 0.5 to 35 MPa in the presence of an oxidic supported catalyst comprising one or more noble metals which has been treated with hydrogen at from 50 to 300°C for at least 0.5 hour before use.

2. A process as claimed in claim 1, wherein the supported catalyst has been treated with hydrogen at from 50 to 300°C for at least 2 hours before use.

3. A process as claimed in claim 1 or 2, wherein the supported catalyst comprises the noble metals palladium and/or platinum and/or ruthenium.

4. A process as claimed in any of claims 1 to 3, wherein the supported catalyst contains from 0.1 to 5% by weight of palladium and from 0.01 to 5% by weight of platinum.

5. A process as claimed in any of claims 1 to 4, wherein the support used for the catalyst is zirconium dioxide or aluminum oxide.

6. A process as claimed in any of claims 1 to 5, wherein the amine used is dimethylamine.

7. 1,5-Bis(dimethylamino)-2-methylpentane.

## Revendications

1. Procédé pour la préparation des 2-méthyl(1,5-pentanediamines secondaires ou tertiaires de formule I 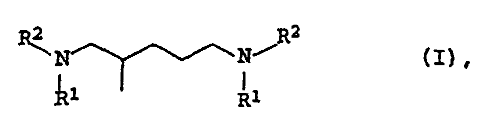 dans laquelle
R¹ et R² représentent un atome de H, un groupe alkyle en C₁ à C₂₀, cycloalkyle en C₃ à C₈, aryle ou arylalkyle en C₇ à C₂₀,
dans laquelle les restes R¹ et R² présentent éventuellement des substituants choisis dans le groupe formé par les groupes alkyle en C₁ à C₂₀, alcoxy en C₁ à C₂₀, aryloxy en C₆ à C₂₀ et hydroxy, et dans laquelle R¹ et R² ne représentent pas en même temps un atome d'hydrogène,
ou
R¹ et R² représentent conjointement une chaîne alcène en C₃ à C₇ qui est éventuellement substituée par un groupe alkyle en C₁ à C₂₀ et/ou un groupe alcoxy en C₁ à C₂₀, et qui est éventuellement interrompue, une ou deux fois, par un atome de O ou par NR³, et
R³ représente un atome de H ou un groupe alkyle en C₁ à C₂₀,
procédé dans lequel on met à réagir le dinitrile 2-méthylglutarique avec une amine primaire ou secondaire de formule R¹R²NH et l'hydrogène, à une température comprise entre 50°C et 250°C et à une pression comprise entre 0,5 Mpa et 35 Mpa, en présence d'un catalyseur d'oxyde sur support contenant un ou plusieurs métaux nobles et que l'on a hydrogéné pendant une durée d'au moins 0,5 heure à une température comprise entre 50°C et 300°C, avant de le mettre en oeuvre.

2. Procédé selon la revendication 1, dans lequel l'on traite le catalyseur sur support hydrogéné pendant une durée d'au moins 2 heures à une température comprise entre 50°C et 300°C, avant de le mettre en oeuvre.

3. Procédé selon les revendications 1 et 2, dans lequel le catalyseur sur support contient, en tant que métaux nobles, le palladium et/ou le platine et/ou le ruthénium.

4. Procédé selon les revendications 1 à 3, dans lequel le catalyseur sur support contient le palladium à raison de 0,1% à 5% en poids et le platine à raison de 0,01% à 5% en poids.

5. Procédé selon les revendications 1 à 4, dans lequel l'on met en oeuvre, en tant que support du catalyseur, le dioxyde de zirconium ou l'oxyde d'aluminium.

6. Procédé selon les revendications 1 à 5, dans lequel on met en oeuvre, en tant qu'amine, la diméthylamine.

7. Le 1,5-bis-(diméthylamino)-2-méthyl-pentane.
